Europäisches Patentamt

European Patent Office

Office européen des brevets

(11) Publication number: **0 505 908 A1**

## EUROPEAN PATENT APPLICATION

(21) Application number: **92104656.1**

(22) Date of filing: **18.03.92**

(51) Int. Cl.⁵: **C12N 15/62**, C12N 15/12,
C12N 15/13, C12N 1/21,
C12N 5/10, C07K 13/00,
A61K 37/02

The microorganism(s) has (have) been deposited with DSM under number(s) DSM 6370 and DSM 6369.

(30) Priority: **27.03.91 EP 91810220**

(43) Date of publication of application:
**30.09.92 Bulletin 92/40**

(84) Designated Contracting States:
**AT BE CH DE DK ES FR GB GR IT LI LU MC NL PT SE**

(71) Applicant: **F. HOFFMANN-LA ROCHE AG
Postfach 3255
CH-4002 Basel(CH)**

(72) Inventor: **Karjalainen, Klaus
12 Andreasplatz
CH-4051 Basel(CH)**
Inventor: **Lanzavecchia, Antonio
17 Augustinergasse
CH-4051 Basel(CH)**
Inventor: **Traunecker, André
11 rue du Moulin
F-68450 Wolschwiller(FR)**

(74) Representative: **Mezger, Wolfgang, Dr. et al
Grenzacherstrasse 124 Postfach 3255
CH-4002 Basel(CH)**

(54) **Chimaeric polypeptides.**

(57)    The present invention relates to a DNA sequence comprising two DNA subsequences, each subsequence-independently from the other one-coding for a ligand binding site of a human receptor, an antigen binding site or a ligand of a human receptor and optionally further comprising a third DNA subsequence coding for the constant domain or parts thereof of a human immunglobulin $x$-or $\lambda$-light chain, to vectors containing such DNA sequences, to prokaryotic or eukaryotic host cells transformed with such vectors, to polypeptides encoded by such DNA sequences, to a method for the production of such polypeptides, to pharmaceutical compositions containing such polypeptides and to the use of such polypeptides for the preparation of pharmaceutical compositions, especially compositions for the treatment of AIDS and the use of such polypeptides for the treatment of deseases, especially AIDS.

Rank Xerox (UK) Business Services

Since the realization that the human immunodeficiency virus type I (HIV-I) exploits the cell surface CD4 molecule as its receptor, soluble forms of the human CD4 (sCD4) molecule have been prepared to combat the virus (1-4). The first type of sCD4 molecules contained basically only the extracellular part of the protein or truncated versions of it and, thus, were able only to neutralize the free virus but not to kill virus infected cells (5-9). Further development led to molecules like CD4-toxin (10) and CD4-Igs (Immunglobulin chains) (11,12) which were constructed to destroy the infected cells by direct action of the toxin or by Fc mediated immune effector functions.

Such CD4 based molecules have also been the subject of various patent applications, e.g. WO 89/03222 filed by the Dana-Faber Cancer Institute, WO 88/01304 filed by The Trustees of Columbia University in the City of New York, WO 89/01940 filed by Biogen, Inc., WO 89/02922 and European Patent Application Publ. No. (EPA) 314 317 filed by Genentech., Inc., and EPA 325 262 filed by The General Hospital Corporation and EPA 394 827 filed by Hoffmann-La Roche.

It is an object of the present invention to provide new CD4 based chimaeric polypeptides. These polypeptides comprise the first two domains of the human CD4 molecule or fragments thereof with intact ligand binding activity and an antigen binding site which recognizes the human T-cell receptor complex of cytotoxic T-cells (CD3-molecule) and optionally further comprises the constant domain or parts thereof of an immunglobulin $x$ or λ-light chain which can be of any origin, preferentially mammalian, e.g. mouse or rat. The expression "antigen binding site" (Fv) means in the context of the present invention an amino acid sequence which comprises the binding portions of the variable domain of the light and the heavy chain of an antibody and a spacer between these two binding portions. Binding portion means an amino acid sequence comprising at least a part of the variable domain of the light or the heavy chain of an antibody preferentially the whole variable domain of the light or the heavy chain of an antibody so that the corresponding antigen will still be bound when these binding portions of the light and the heavy chain are both present in a molecule. In a specific embodiment of the present invention binding portion means an amino add sequence consisting of the whole variable domain of the heavy chain of an antibody and some, preferentially four additional amino acids, e.g. Gly-Val-His-Cys, in front of the N-terminus. The number and type of amino acids of the spacer can be choosen by a man skilled in the art on the basis of the considerations given by Huston et al (16). Finally an abbreviation in the form of "FvX" means in the context of the present invention an antigen binding site of an anti-"X"-antibody, e.g. "FvCD3" means an antigen binding site of an anti-CD3-antibody.

Preferred are such chimaeric polypeptides which comprise beside FvCD3 the first two domains of the human CD4 molecule, preferentially with the following amino acid sequence (amino acids are given by the standard one letter code):

K K V V L G K K G D T V E L T C T A S Q K K S I Q F H W K N S N Q I K I L G
N Q S F L T K G P S K L N D R A D S R R S L W D Q G N F P L I I K N L K I E D
S D T Y I C E V E D Q K E E V Q L L V F G L T A N S D T H L L Q G Q S L T L T
L E S P P G S S P S V Q C R S P R G K N I Q G G K T L S V S Q L E L Q D S G T
W T C T V L Q N Q K K V E F K I D I V V L

Further preferred polypeptides of the present invention are chimaeric CD4 based polypeptides as mentioned above whereby the FvCD3 part is derived from an anti-CD3-antibody secreted by the hybridoma "TR66" (15).

Such CD4-based chimaeric polypeptides show considerable advantages over the already known CD4-Toxin or CD4-Ig constructs, because they are able to mediate a more potent killing of the HIV-I virus infected cells and also because activated T-cells may provide efficient transport of these molecules to sites of infection. In addition their simple structure (e.g. low molecular weight) makes them suitable for prokaryotic expression [see e.g. Tai et al. (34)].

In order to mediate direct killing of other target cells by cytotoxic T-cells the CD4-part of above mentioned molecules can be replaced by any other molecule recognizing a target cell specific surface protein. Such other target cell recognizing molecule can be a ligand binding site of a human receptor, e.g. a soluble part of a receptor, like in the case of CD4, or a ligand of a human receptor which is specific for the target cell, e.g. Il-7 for IL-7 receptor positive cells or human stem cell factor for human stem cells or another antigen binding site recognizing an antigen specific for the desired target cell, e.g. FvclassII (specific for class II positiv cells) or an antigen binding site recognizing a tumor cell specific antigen, like in the case of Fvovca (antigen derived from ovary carcinoma cells) or an antigen binding site recognizing a virus specific surface antigen, e.g. Fvmol recognizing the mouse molony leukemia virus surface protein "gp70" as their corresponding antigen. The expression "ligand binding site of a human receptor" means in the context of the present invention an amino acid sequence which folds in a manner that the ligand of the corresponding human receptor can be bound like this is for example the case for what is understood by a man skilled in the art by the expression "soluble part of a receptor".

However in order to direct to a desired target cell another activity beside killing via cytotoxic T-cells the FvCD3 part can be replaced by any other molecule recognizing a cell or molecule which can create or mediate such an activity. Such a recognizing molecule can be again a soluble part of a receptor recognizing a surface protein of the cell of interest or a ligand of a human receptor specific for such a cell or an antigen binding site recognizing an antigen specific for the desired cell or the molecule creating or mediating the desired activity. For example chimaeric polypeptides comprising a part recognizing a surface protein of a virus, whereby such part is e.g. in the form of an antigen binding site, e.g. Fvmol and the other part of the chimaeric polypeptide recognizing a desired target cell whereby such other part is e.g. in the form of a ligand of the receptor specific for this target cell e.g. human stem cell factor (SCF; the ligand of ckit) can be constructed. In such a way a virus transformed by foreign DNA can be directed to cells of interest, e.g. in this case stem cells, for somatic gene therapy. It is therefore another object of the present invention to provide such chimaeric polypeptides.

Since chimaeric polypeptides as described above can behave in some aspects like bispecific antibodies especially in case where such chimaeric polypeptides comprise two different antigen binding sites it is accordingly understood that the chimaeric polypeptides of the present invention can be used for the same purposes as such bispecific antibodies.

However the chimaeric polypeptides of the present invention show at least the following considerable advantages over functional comparable bispecific antibodies. The usual approach to generate bispecific antibodies is to fuse two hybridoma cell lines producing the desired antibody specifities (17). Because of the random assortment of Ig heavy and light chains, these hybridomas produce a potential mixture of 10 different antibody molecules. Therefore, the bispecific antibodies have to be purified with cumbersome procedures which can considerably lower the yield of the desired product. In the present case chimaeric polypeptides are produced as a single chain molecule. In addition such molecules show a considerable lower molecular weight than bispecific antibodies. This is an advantage for their production in prokaryotic cells and for the penetration to desired target regions when used for therapeutic or diagnostic purposes.

Dimeric bispecific analogs of CD4-H$\gamma$3 (disclosed in EPA 394 827) consisting of one monomer in the form of CD4-H$\gamma$3 and the other in the form of FvCD3-H$\gamma$3 (CD4-H$\gamma$3/FvCD3-H$\gamma$3) are also an object of the present invention. Such molecules wherein the CD4-part and/or FvCD3 is replaced by a ligand binding site of a receptor, another antigen binding site than FvCD3 or a ligand of a human receptor whereby CD4-H$\gamma$3 is excluded are also an object of the present invention. On the basis of their structural features such molecules are comparable in function to bispecific antibodies which are however more cumbersom to produce (see above) as the bispecific H$\gamma$3-constructs (instead of 10 different antibody molecules in case of bispecific antibodies only 3 different ones are generated).

It is therefore an object of the present invention to provide a DNA sequence comprising two DNA subsequences, each subsequence-independently from the other one-coding for a ligand binding site of a human receptor, an antigen binding site or a ligand of a human receptor and optionally further comprising a third DNA subsequence coding for the constant domain or parts thereof of an immunglobulin $x$- or $\lambda$-light chain or more specifically to provide such a DNA sequence whereby one of the two DNA subsequences codes for a ligand binding site of a human receptor, preferentially the first two domains of the human CD4 molecule or fragments thereof which still bind the ligand and the other DNA subsequence codes for an antigen binding site. A specifically preferred such DNA sequence comprises a DNA subsequence coding for the first two domains of the human CD4 molecule or fragments thereof which still bind the ligand whereby a sequence coding for the first two domains of human CD4 molecule are preferred and a second DNA subsequence coding for an antigen binding site which recognizes the human CD3 molecule. Under parts of the first two domains of the human CD4-molecule the following possibilities are understood:

(a) the complete first domain in combination with parts of the second domain, or

(b) the complete second domain in combination with parts of the first domain, or

(c) a combination of parts of either domain.

It is furthermore an object of the present invention to provide such a DNA sequence comprising two DNA subsequences coding for antigen binding sites with the same or different antigenic specificity whereby antigen binding sites with different antigenic specificities are prefered, especially whereby one such antigen binding site recognizes the human CD3 molecule and optionally further comprising a third DNA subsequence as already specified.

It is furthermore an object of the present invention to provide a DNA sequence comprising a DNA subsequence coding for a ligand of a human receptor and a DNA subsequence coding for an antigen binding site, especially such a site which recognizes the human CD3 molecule and optionally further comprising a third DNA sequence as already specified.

It is furthermore an object of the present invention to provide a vector comprising a DNA sequence as mentioned above, especially such a vector which is a vector capable of replication in prokaryotic and expression in eukaryotic host cells. Furthermore it is an object of the present invention to provide prokaryotic or eukaryotic host cells transformed with such a vector.

In addition it is an object of the present invention to provide chimaeric polypeptides as described already above or as encoded by a DNA sequence as described above, especially as a therapeutically active agent, e.g. for the treatment of AIDS. It is also an object of the present invention to provide a method for the production of such polypeptides which method comprises culturing in a suitable medium a transformed host cell as mentioned above and isolating said polypeptide. The polypeptides of the present invention when ever prepared according to such a method are also an object of the present invention.

Furthermore pharmaceutical compositions containing at least one polypeptide of the invention and therapeutically acceptable carrier materials and the use of the polypeptides of the invention for the preparation of such pharmaceutical compositions, especially such a composition for the treatment of various deseases, e.g. AIDS and the use of the polypeptides of the present invention for diagnostic and therapeutic purposes are an object of the present invention.

The invention will be better understood on the basis of the following detailed description when considered in connection with the accompanying drawings, wherein:

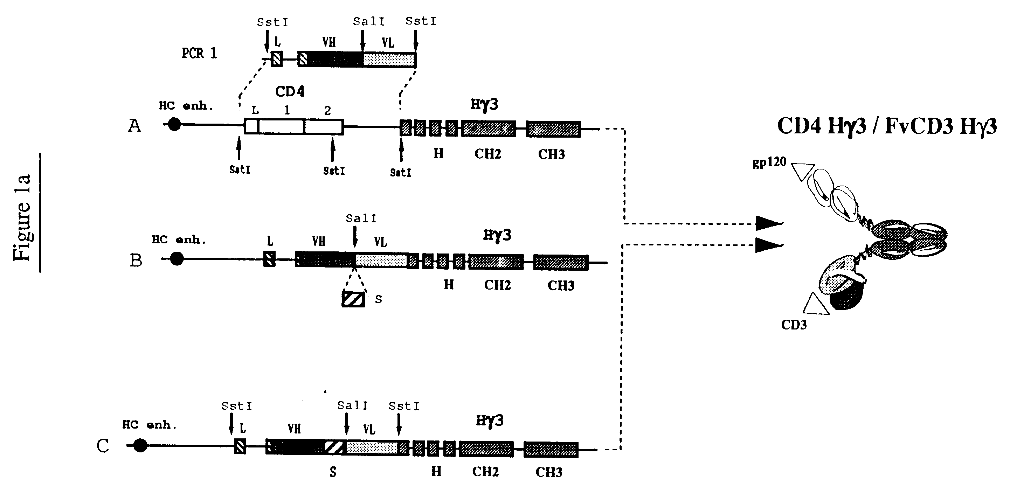

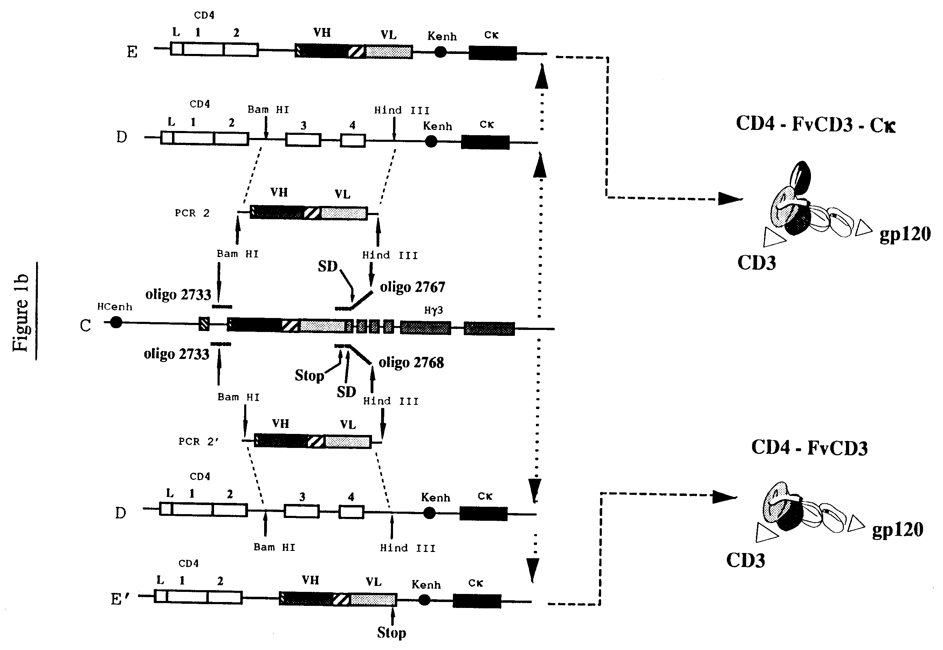

Fig. 1a/Fig. 1b    shows a flow chart of the construction of the final plasmids pFvCD3-H$\gamma$3,pCD4-FvCD3-C$x$ and pCD4-FvCD3 which are partially represented by "C", "E" and "E'", respectively. The corresponding expression products CD4-H$\gamma$3/FvCD3-H$\gamma$3, CD4-FvCD3-C$x$ and CD4-FvCD3 and their ligands CD3 and gp 120 are schematically drawn on the right handside. Intermediate plasmids pCD4-H$\gamma$3,pFvCD3-H$\gamma$3 without the "spacer" between the immunoglobulin variable domains and pHT4-YK12 are partially represented by "A", "B" and "D", respectively. Boxes give coding regions: highly dense dotted boxes refer to the variable domain of the heavy chain of the monoclonal antibody "TP66" ("VH"); striped boxes (stripes from left top to right bottom) indicated by "L" representing the two parts of the leader sequence resulting from the antibody "SP6"; less dense dotted boxes in connection with "VL" represent the variable domain of the light chain of the monoclonal antibody "TR66"; more intensely dotted boxes indicated by "H", "CH2" and "CH3" represent the hinge region and the second and third constant domains of the heavy chain of the human IgG3 immunoglobulin ["H$\gamma$3"]; open boxes indicated by "L", "1", "2", "3", "4" in connection with CD4 represent the leader sequence and the corresponding domains of the human CD4 molecule; a striped box (stripes from left bottom to right top) represent the linker "S" between the two variable immunglobulin domains; a dotted box indicated by "C$x$" represents the constant domain of the mouse $x$-light chain. Filled circles represent in case of "HC-enh." the immunglobulin heavy chain enhancer and in case of "Kenh." the immunglobulin $x$-light chain enhancer. "PCR1" and "PCR2" represent the result of the corresponding PCR reactions. "Oligo" represents oligonucleotides and "SD" and "stop" represent a splice donor site and a stop codon. "SstI", "SalI", BamHI" and HindIII refer to the corresponding restriction sites (standard abbreviations).

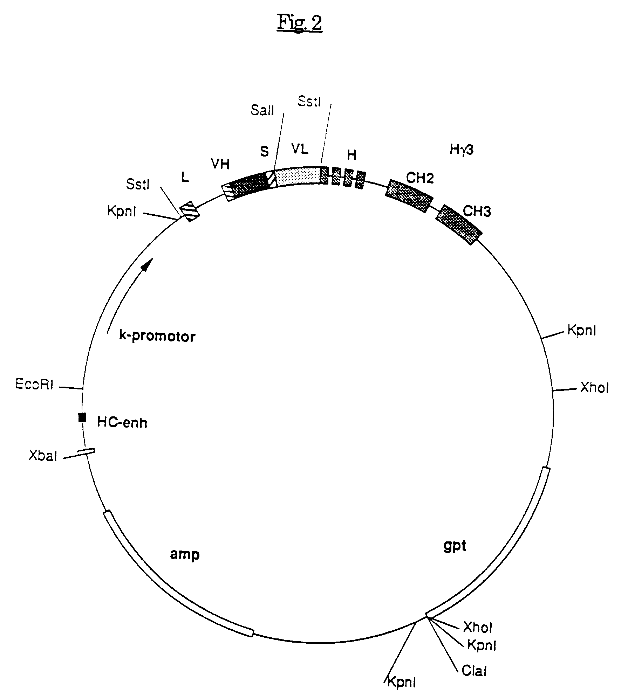

Fig. 2    shows a schematic drawing of pFvCD3-H$\gamma$3 whereby "K-promoter" refers to the immunglobulin $x$ light chain promoter, "amp" to the ampicillin resistance gene, "gpt" to the E.coli xanthine guanine phosphoribosyl transferase gene and "KpnI", "XhoI", "ClaI", "XbaI" and "EcoRI" to the corresponding restriction sites given by standard abbreviations and all the other abbreviations and symbols are as specified for Figure 1.

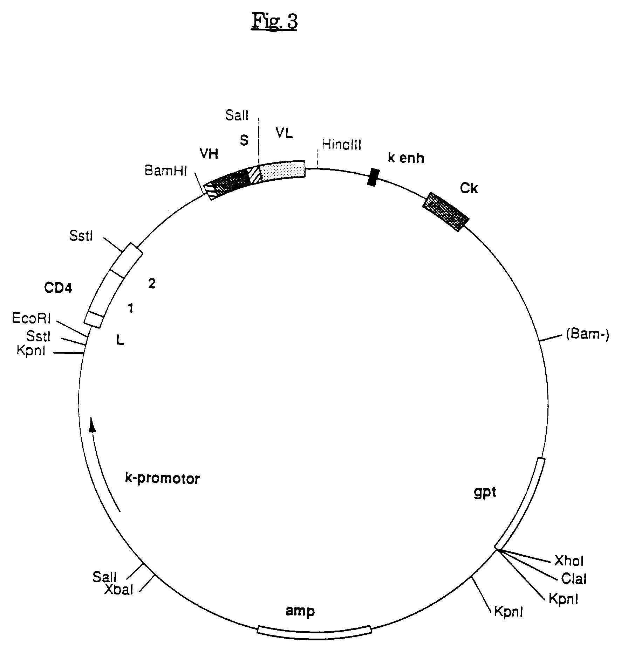

Fig. 3    shows a schematic drawing of pCD4-FvCD3-C$x$ whereby the abbreviations and symbols are as specified for Figure 1 or Figure 2.

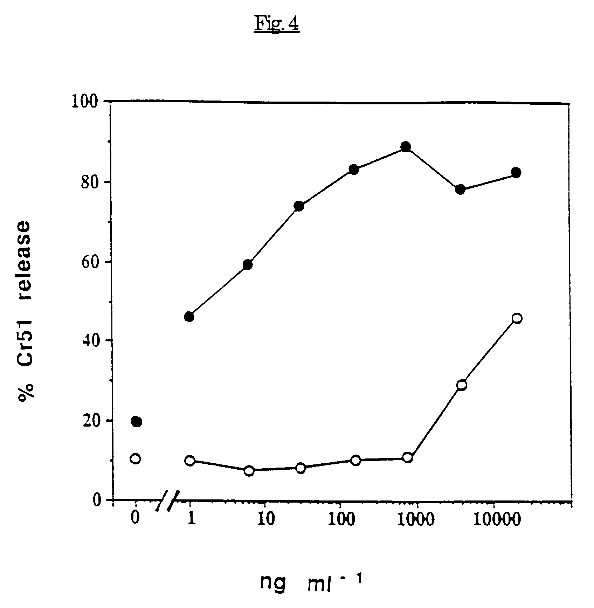

Fig. 4    shows the percentage of Cr$^{51}$ release dependent on the amount of CD4-FvCD3-C$x$ indicated in ng ml$^{-1}$ as a result of the assay as described in detail in Example 3 whereby open circles represent not infected and filled circles represent HIV-I infected Jurkat cells.

DNA sequences coding for antigen binding sites can be prepared in the form of genomic DNA or cDNA on the basis of a hybridoma cell line secreting an antibody with the desired specificity as the cellular source for the starting genomic DNA or mRNA. Such hybridoma cell lines can be obtained from depository authorities, e.g. the American Type Culture Collection (ATCC) in Rockville, Maryland, USA, like this is the case for example for "OKT3", a hybridoma secreting an anti-CD3-antibody (ATCC No. CRL 8001) or the European Collection of Animal Cell Cultures in Porton Down, Salisbury, United Kingdom. In case that a specific hybridoma cell line is not available such cell line can be produced on the basis of the antigen of interest by methods known in the art and described e.g. by Harlow and Lane (27). For example any human cytotoxic T cell carrying the CD3 receptor molecule can be isolated by methods known in the art and described e.g. by Moretta et al., [J. Expt. Med. 157, 743-754 (1983)] and the cell itself or membrane fractions thereof can be used to generate an anti-CD3 antibody producing hybridoma cell line. Starting from such specific hybridoma cell lines as a source of genomic DNA or mRNA DNA sequences coding for the variable domains of immunglobulin heavy or light chains can be prepared as described for example by Oi et al. (28) or Ochi et al. (29) in the case of genomic DNA sequences and by Neuberger (30) in case of cDNA sequences. DNA sequences which code not only for one of the two variable domains of an immunoglobulin but for both together in form of a single chain molecule can be prepared as described, e.g. in US Patent No. 4,946,778, by Orlandi et al. (18) or by the following method based on the concept of Huston et al. (16) and described for a specific embodiment of the present invention in more detail in Example 1. For such purpose mRNA can be prepared from hybridoma cells as characterized above by methods known in the art (26) or as described e.g. by Chomczynski and Sacchi (22). First strand cDNA synthesis can be prepared by standard methods (26). Second strand synthesis can be performed using a primer specific for the 5'end of the DNA sequence coding for the variable domain of the heavy chain of any immunoglobulin ($V_H$) and a primer specific for the 5'end of the DNA sequence coding for the variable domain of the light chain of any immunoglobulin ($V_L$). In case that DNA sequences coding for the variable domain of the heavy chain and the $x$ light chain of any immunglobulin of mouse origin are concerned 5'-end primers as described by Orlandi et al. (18) can be used. 5'-end primers for DNA sequences coding for the variable domain of λ light chain or of the heavy chain and the $x$ light chain of other origin, like for example rat, rabbit, sheep etc. can be designed on the basis of sequence information as disclosed e.g. by Kabat et al. (31) or any of the known sequence data banks (see below) in a manner as described, e.g. by Orlandi et al. (18). Final amplification by polymerase chain reaction (PCR) can then be achieved by such primers and oligonucleotides complementary to γHC and C$x$-genes of corresponding origin [Kabat et al. (31) or any known sequence data bank (see below)]. In case that such DNA sequences are intended for integration into vectors for eukaryotic expression and secretion, especially in and from hybridoma cells and such vectors do not comprise a leader sequence (L-region) coding for a suitable secretion signal such a leader sequence can be prepared from any suitable eukaryotic gene coding, for a secreted protein on the basis of its known sequence by appropriate oligonucleotides for PCR. For example the L-region from the Sp6-HC gene can be easily amplified by the oligonucleotides as indicated in Example 1 out of plasmid pFvCD3-Hγ3 which has been deposited at the Deutsche Sammlung für Mikroorganismen und Zellkulturen GmbH, Braunschweig, Bundesrepublik Deutschland. All these DNA sequences can then be linked together in one PCR cycle as described for example by Horton et al. (24) employing suitable joining and flanking oligonucleotide probes which have already been used for the amplification of the partial DNA sequences or which can easily be designed by known methods (26) on the basis of information obtained by partial DNA sequencing according to standard methods (26) of the DNA fragments to be linked. The flanking oligonucleotides can be designed in a manner to create suitable restriction sites for the insertion of the resulting DNA sequence into suitable vectors together with other DNA sequences of the present invention for the expression of the chimaeric polypeptides. In the same manner a restriction site can be generated between the DNA sequences coding for the $V_H$ and $V_L$-domains which can be used for the integration of a DNA sequence coding for a suitable spacer. Such DNA sequence can be designed, e.g. as described by Huston et al. (16) and prepared by methods known in the art (26).

DNA sequences coding for a ligand binding site of a human receptor, e.g. the soluble part a human receptor or a DNA sequence coding for a human ligand of a receptor of interest can be prepared on the basis of known partial or total amino acid or nucleic acid sequence information by standard methods (26), preferentially by the known PCR-technology [see e.g. references given in Example 1 or "PCR Protocols" edt. by M.A. Innis, D.H. Gelfand, J.J. Sninsky and T.J. White, Academic Press, San Diego]. Such sequence information can be obtained in principle from any sequence data base, for example, like Genbank (Intelligenetics, California, USA), EMBL (Heidelberg, FRG), NBRF (Georgetown University, Medical Centre, Washington DC, USA) and Vecbase (University of Wisconsin, Biotechnology Centre, Madison, Wisconsin, USA) or more specifically for example for the human CD4-molecule from Maddon et al. (13), for human IL-7 from Namen, A. et al. (35) and for the human stem cell factor from Martin et al. (32). A DNA sequence coding for the first two domains of the human CD4 molecule or fragments thereof which still bind the corresponding ligand can be prepared as stated above or more specifically as described in EPA 394 827 wherein also plasmids which have been deposited at the DSM are described which can be used as a source for such DNA sequence.

DNA sequences of the present invention obtained as described above can then be handled and integrated into suitable vectors according to methods well known in the art (26). It is well understood and covered by the present invention that DNA sequences of the present invention may comprise parts of different origin, viz. parts of cDNA and parts of genomic origin.

Suitable vectors for the practice of the present invention comprise vectors which are known in the art, especially such vectors which can be used for the expression of polypeptides in eukaryotic host cells like, e.g., pSV2 derived vectors [described for example by German, C. in "DNA Cloning" (14)] like pSV2-cat [ATCC No. 37 155], pSV2-dhfr [ATCC No. 37146], pSV2-neo [ATCC No. 37149] or pSV2-gpt [ATCC No. 37145] or pSV2-gpt derived vectors, like pHT4-Y1, pCD4-M$\gamma$2a, pCD4-M$\mu$, pCD4-H$\mu$, pCD4-H$\gamma$1 and pCD4-H$\gamma$3 whereby the latter five vectors have been deposited at the Deutsche Sammlung von Mikroorganismen und Zellkulturen GmbH (DSM) in Braunschweig, BRD, at the 26th of January 1989 under accession numbers DSM 5173, DSM 5174, DSM 5315, DSM 5314 and DSM 5523, respectively and are described in EPA 394 827. Especially preferred pSV2-gpt derived vectors are pFvCD3-H$\gamma$3 and pCD4-FvCD3-C$_x$ which have been deposited under the Budapest Treaty for patent purposes at the DSM in Braunschweig, BRD at the 18th of February 1991 under accession numbers DSM 6370 and DSM 6369 respectively.

Further preferred are vectors which can be used for the expression of polypeptides in prokaryotic host cells, like for example E.coli. For this purpose DNA sequences of the present invention have to be modified by methods known in the art (26) in order to make them suitable for expression in prokaryotic host cells, e.g. E.coli. Suitable vectors used for expression in E.coli are mentioned e.g. by Sambrook et al. (26) or by Fiers et al. in "Procd. 8th Int. Biotechnology Symposium" [Sec. Franc. de Microbiol., Paris, (Durand et al., eds.), pp. 680-697 (1988)] or vectors of the pDS family [Bujard et al., Methods in Enzymology, eds. Wu and Grossmann, Academic Press, Inc. Vol. 155, 416-433 (1987); "System for high level production in E.coll and rapid purification of recombinant proteins: application to epitope mapping, preparation of antibodies and structure function analysis" by Stüber et al. in Immunological Methods, Vol. IV, Lefkovits and Pernis (eds.), Academic Press, New York (in press)].

Prokaryotic or eukaryotic host cells can then be transformed by such vectors. Suitable prokaryotic host cells for such purpose are well-known to a person skilled in the art and include bacteria, for example, E.coli, especially E.coli K12 strains, e.g. M15 [described as DZ 291 by Villarejo et al. (20)] and HB 101 [ATCC No. 33694].

Suitable eukaryotic host cells also well known to persons skilled in the art (26) include cells of vertebrate, yeast or other origin with myeloma cell lines like,e.g., P3X63Ag8 [ATCC No. TIB9] being preferred. In accordance with the specific host cell to be used in the practice of the present invention suitable promotor-elements like for example the Ig heavy chain promotor or the Ig$x$-promotor (see Example 1) and if desired suitable enhancer elements like for example the Ig heavy chain enhancer or the Ig light chain enhancer (see Example 1) can be integrated into the plasmids used for the transformation of such host cells by methods well known in the art (26).

Transformation of the host cells by vectors as described above may be carried out by any conventional procedure (26) or by the specific procedures as described below or in Example 2.

Where the host cell is a prokaryotic cell such as E.coli for example, competent cells which are capable of DNA uptake are prepared from cells harvested after exponential growth phase and subsequently treated according to the known CaCl$_2$-method.

Where the host used is an eukaryotic cell the following transfection methods like calcium phosphate precipitation, the DEAE-Dextran method [see also C. Gorman in "DNA Cloning", (14)], conventional mechanical procedures such as microinjection, insertion of a plasmid encapsulated in red blood cell hosts or in liposomes, treatment of cells with agents such as lysophosphatidylcholin, use of virus vectors or protoplast fusion can be employed in the practice of the present invention, with protoplast fusion being a preferred method (see Example 2).

Transformed cells can then be selected and cultivated according to methods well known in the art. For example cells which have been transformed by a vector carrying a "neo" gene coding for a phosphotransferase can now be cultured in the presence of the drug G418 or in case of a vector carrying a "gpt" gene transformed cells can be grown in specific media containing xanthine and mycophenolic acid (Gorman in "DNA Cloning", Vol. II, see above).

Chimaeric polypeptides of the present invention produced by transformed cells as stated above can be recovered by any appropriate method known in protein and peptide chemistry such as, for example, precipitation with ammonium sulfate, dialysis, ultrafiltration, gelfiltration or ion-exchange chromatography, gel electrophoresis, isoelectric focusing, affinity chromatography, like immunoaffinity chromatography, HPLC or the like, whereby affinity chromatography is preferred. For example chimaeric polypeptides of the present invention comprising a receptor binding site can be purified by affinity chromatography, whereby the corresponding ligand is fixed to an appropriate matrix according to methods known in the art [see e.g. as described in a guide on affinity chromatography published by Pharmacia, Uppsala, Sweden] or in the opposite way where such a chimaeric polypeptide comprises the ligand itself by an affinity matrix to which the corresponding receptor binding site has been fixed. In case such chimaeric polypeptides comprise at least one antigen binding site purification is possible by affinity chromatography employing the corresponding antigen. Finally such polypeptides can be easily purified by immunaffinity chromatography. For example chimaeric polypeptides comprising a part of the human CD4 molecule can be purified by gp 120-affinity chromatography or immunaffinity chromatography using an anti-CD4-antibody [Becton Dickinson AG, Basle, CH] which can be linked to a suitable matrix for such purpose as known in the art [see e.g. Guide on affinity chromatography edt. by Pharmacia, Uppsala, Sweden]. In case that any of the chimaeric polypeptides of the present invention comprise a mouse constant region of the $x$ light chain such polypeptides can be purified by immunaffinity chromatography using an antibody against the constant domain of the $x$-light chain of a mouse immunglobulin [see Yelton et al. (19)] or such a polypeptide comprises $H\gamma3$ it can be isolated by Protein G-chromatography [Pharmacia, Uppsala, Sweden] or immunaffinity chromatography using an anti human immunglobulin G heavy chain antibody isolatable according to standard methods (27) from a corresponding myeloma cell line [ATCC No. HB60].

The chimaeric polypeptides of the present invention can be characterized in the following manner. In case such polypeptides comprise the first two domains of the human CD4 molecule or fragments thereof which still bind the ligand they can be characterized according to their binding to "gp120" the surface protein of the HIV-I virus by methods known in the art and described e.g. in EPA 394 827. Such polypeptides comprising in addition also FvCD3 can be characterized by their ability to bind to $CD3^+$ cells by methods known in the art, e.g. ($CD3^+$, $CD4^-$)-T cells or cell lines [see e.g. Lanzavecchia and Scheidegger (15)] can be incubated with such chimaeric polypeptides, washed and further incubated with an anti-CD4-antibody followed by a FITC-labelled anti-immunglobulin antibody (FITC = fluorescein isothiocyanate) and analyzed on a fluorescent activated cell sorter. In general polypeptides of the present invention comprising FvCD3 and another antigen binding site, or a ligand binding site of a human receptor or a ligand of a specific target cell can be characterized by an assay measuring cytotoxic T cell mediated target cell killing, e.g. as described in Example 3 incase of CD4-FvCD3-$C_x$ for HIV infected target cells expressing "gp120" to which the CD4 part of the molecule binds. For such assay any human cytotoxic T cell or T cell clone, isolatable e.g. according to Moretta et al. (s.a.) can be used. In addition any HIV-virus infected human cell or cell line beside the well known Jurkat cell line can be used for such an assay. It should however, be noted that some human tumor cells, like for example H9 cells seem to be recognized and accordingly killed in culture directly by cytotoxic T cells in the absence of chimaeric polypeptides of the invention and can therefore not be used for such an assay. In principle any HIV-I virus isolate which has the ability to infect a given target cell which expresses after infection the gp120-molecule, such as for example: HTLV-IIIB, obtainable from infected H9 cell lines (ATCC No. CRL 8543) by methods known in the art, or for example as described by Popovic et al. (21) or LAV (CNCM No. I-232) can be used in such an assay. Infection by such an HIV-I isolate of cells or cell lines can be done as known in the art. As shown in Figure 4 only ng amounts of CD4-FvCD3-$C_x$ were sufficient for efficient killing of HIV-I infected Jurkat cells. At higher concentrations ($\approx 1 \mu g/ml$), noninfected cells were also killed, because at these concentrations CD4-FvCD3-$C_x$ start to dimerize due to their $C_x$ moieties. Dimeric CD4-FvCD3-$C_x$, in fact, bridge the CD3 molecules of cytotoxic T cell clones to the CD3 molecules of Jurkat cells and induce killing of uninfected cells. In case that higher concentrations of CD4-FvCD3-$C_x$ are desirable, CD4-FvCD3-$C_x$ can be replaced by CD4-FvCD3.

In accordance with the present invention the novel chimaeric polypeptides of the present invention may be used in the treatment of various deseases. For example such chimaeric polypeptides comprising the first two domains of the human CD4-molecule or fragments thereof, e.g. CD4-FvCD3-$C_x$ or CD4-FvCD3, can be used in the treatments of AIDS. In general all such polypeptides comprising FvCD3 can be used for the killing of target cells which influence the human body in a not desirable fashion, like for example cancer cells in case that the second part of the chimaeric molecule recognizes such type of cancer cell. For example FvCD3-Fvovca can be used for the killing of ovary carcinoma cells. In case that a chimaeric polypeptide of the present invention comprises beside FvCD3 an antigen, T cell help may be concentrated into relevant antigen specific B-cells and therefore such chimaeric polypeptides may behave as "super immunogens". In case of chimaeric polypeptides comprising Fvmol thereby being capable of recognizing a surface protein of the mouse malony leukemia virus, any DNA sequence which can be integrated into such a virus (J.R. McLachlin et al. in Progress in Nucl. Acd. Res. and Mol. Biol., edt. by W.E. Cohn and K. Moldave, Vol. 38, Academic Press, San Diego), can be transformed into a target cell which is recognized by the second part of the chimaeric molecule, like this can be the case for stem cells for Fvmol-SCF and used accordingly for somatic gene therapy.

Such chimaeric polypeptides may be administered in pharmaceutically acceptable forms, especially in an injectable form. Dosage and dose rates may parallel that currently being used in clinical applications of other known polypeptides of comparable structure and/or function. Pharmaceutical compositions may contain at least one chimaeric polypeptide of the present invention in association with pharmaceutically acceptable solid or liquid carrier materials. Any conventionally used carrier material can be utilized. Furthermore, the pharmaceutical compositions may contain other pharmaceutically active agents and be prepared by methods known in the art.

Having now generally described the invention, the same will become better understood by reference to the specific examples which are included herein for purpose of illustration only and are not intended to be limiting unless otherwise specified.

Examples

Example 1

Construction of Plasmids

Plasmid constructions were carried out as described in the following paragraphs. In case that no specific references or details of preparation are given standard methodology according to Sambrook et al. (26) was used.

Plasmid pFvCD3-H$_\gamma$3 (Fig. 1, "C"; Fig. 2) comprising a DNA sequence coding for the variable regions of the heavy and the light chain ("V$_H$ and V$_L$") of an anti-human CD3 antibody (FvCD3) derived from hybridoma "TR66" (15) and for all domains except the first of the constant regions of the heavy chain of the human immunglobulin G3 was constructed in the following manner.

First DNA sequences coding for the V$_L$- and V$_H$-domains of the anti-CD3 hybridoma TR66 were amplified without their leader (L) sequences by using the established PCR methodology (18) with the following modifications. RNA from TR66-cells was extracted by using the guanidium thiocyanate method (22) and the first strand cDNA synthesis was made with M-MLV (BRL Life Technolgies, Gaithersburg, MD, USA) reverse transcriptase and oligo dT primers using standard conditions. The second strand synthesis was performed by T4 DNA polymerase and 5' oligonucleotides "1570" for V$_H$ and 1569 for V$_L$ sequences (18) in conditions described for site directed mutagenesis (Muta-Gene-Protocol, Bio-Rad, Richmond, CA, USA). Final amplification was then achieved by using the above mentioned oligonucleotides and 3' oligonucleotides "1354" and "1329" complementary to mouse $\gamma$HC (immunglobulin-$\gamma$-heavy chain) and C$x$ - (constant domain of immunglobulin $x$-light chain) genes, respectively at standard PCR conditions (30 cycles) recommended by the manufacturer (Gene Amp. DNA amplification Kit, Perkin Elmer Cetus). The L region (initiation codon and leader sequence for eukaryotic secretion) of the Sp6 HC gene [(23); EMBL Gene Bank, Accession No. X56936] containing the small intron was amplified by using 5' oligonucleotide "1609" and 3' oligonucleotides "1610" which are also complementary (20bp) to oligonucleotides "1570" at its 5' end (i.e. to the beginning of the amplified V$_H$ fragment). Partial DNA sequencing of the amplified V$_H$ and V$_L$ fragments showed that they contained JH2 and J$x$5 regions (immunglobulin joining regions of a heavy chain in case of H2 and a light chain in case of $x$5), respectively. Based on this information appropriate junctional and flanking oligonucleotides were designed and used to link the L region from Sp6, V$_H$ and V$_L$ fragments together in one 35 cycle PCR reaction (24): the flanking 5' and 3' oligonucleotides were "1609" and "2768", respectively, the latter having complementarity (24pb) to the end of the mouse J$x$5-sequence. The oligonucleotide "2139" which links V$_H$ to V$_L$ fragments had 20bp complementarities to the end of the JH2-sequence and to oligonucleotide "1569", i.e. to the 5' end of the VL sequence. In addition, the SalI restriction site was designed between these regions of complementarity. All the amplified fragments (Sp6-L, V$_H$, V$_L$) were mixed in equimolar ratios while the flanking oligonucleotides were in 50 fold excess to the linking oligonucleotide during the PCR reaction. Note that no linking oligonucleotide was necessary between Sp6L and V$_H$ fragments because their primary amplification resulted already in complementary ends. The full length product (Fig. 1, PCR1) was then isolated and digested with SstI restriction enzyme (flanking oligonucleotides contained terminal SstI sites) and cloned into similarly digested pCD4-H$_\gamma$3 (Fig. 1, "A") [DSM 5523; described in EPA 394 827]. Finally, a synthetic piece of DNA with SalI compatible ends (oligonucleotides "2119" and "2120"), corresponding to the linking peptide between V$_H$ and V$_L$ domains, was cloned into a SalI site of this intermediate plasmid (Fig. 1, "B") to give plasmid pFvCD3-H$_\gamma$3. The extra amino acids thus introduced between V$_H$ and V$_L$ domains in the final protein have the amino acid sequence: VEGGSGGSGGSGGSGGVD (indicated by the standard one letter code).

Plasmid pCD4-FvCD3-C$_x$ (Fig. 1, "E"; Fig. 3) comprising a DNA sequence coding for the first two domains of human CD4, FvCD3 and the constant region of an immunglobulin $x$ light chain (C$_x$) was constructed in the following manner. DNA coding for FvCD3 was amplified by PCR technology out of pFvCD3-H$_\gamma$3 by using oligonucleotide pair "2733" which brought in the compatible Bam HI restriction site and "2767" which brought in the splice donor site. This fragment (Fig. 1; "PCR2") was then cloned into pHT4-YK12 (Fig. 1, "D") [(12); see also EPA 394 827] as indicated in Figure 1 to give plasmid pCD4-FvCD3-C$_x$.

Plasmid pCD4-FvCD3 (Fig. 1,"E'") coding for the first two domains of the human CD4 molecule and FvCD3 was constructed in the same manner as plasmid pCD4-FvCD3-C$_x$. However, in case of oligonucleotide "2767" oligonucleotide "2768" creating a stop codon was used.

The following oligonucleotides were used for the construction of above mentioned plasmids (restriction sites are indicated by standard abreviations above or below the corresponding oligonucleotide; "SD" means splice donor site; "Stop" means stop codon):

"1570":  AGGTCAAACTGCAGCAGTCAGG 3'
         GC  G         G     T

"1569":  GACATTCAGCTGACCCAGTCTCCA 3'

"1354":  AAATAGCCCTTGACCAGGCATCC 3'

"1329":  GTTAACTGCTCACTGGATGG 3'

"1609":  AGTGTTCCTCTCTACA<u>GAGCTC</u>CTGACAACACTGACTC 3'
                         SstI

"1610":  CCTGACTGCTGCAGTTTGACCTGGCAATGGACACCTAT 3'
         A       C       C GC

"2139":   TGGAGACTGGGTCAGCTGAATGTCGTCGACTGAGGAGACTGTG   <u>SalI</u>
AGAGTGGTGCC 3'

"2768":   AAGTGGGGTTTT<u>GAGCTC</u>TTTCAGC TCCAGCTTGGTCCCAGC 3'
                    SstI

"2119":   TCGAAGGTGGAAGTGGAGGTTCTGGTGGAAGTGGAGGTTCAGG
TGGAG 3'

"2120":   TCGACTCCACCTGAACCTCCACTTCCACCAGAACCTCCACTTCCA
CCT 3'

"2733":   AATCTGAATT<u>GGATCC</u>AGGGCCTGAGGTGAGA 3'
                BamHI

"2767":   TGTACTTACGTTTCAGCTCCAGCTTGGTCCCAGC 3'
        SD

"2768":   TGTACTTACGCTATTTCAGCTCCAGCTTGGTCCCAGC 3'
          Stop

Example 2

Expression and purification of chimaeric polypeptides

Maintenance of animals cells and cell-lines, cloning-methodology, selection procedures and expansion of cloned cells were carried out using standard methodology as described by Freshney, R.I. in "Culture of animal cells: A manual of basic technique" (25).

J558L cells [Oi et al. (28)] were stably transfected with the plasmid pCD4-FvCD3-Cx by protoplast fusion as described by Oi et al. (28).Transfectants were selected by including into the basic medium (Dulbecco's modified Eagle's medium, 10% fetal calf serum (FCS), 5 x $10^{-5}$ M 2-mercaptoethanol) 5 $\mu$g/ml mycophenolic acid and 250 $\mu$g/ml xanthine according to Traunecker et al. (33). Transfection frequencies were in the range of $10^{-5}$ to $10^{-4}$.

Secreted CD4-FvCD3-Cx was purified in a single step by immunaffinity chromatography (see "Affinity Chromatography" edt. by Pharmacia, Uppsala, Sweden) using an anti-Cx-monoclonal antibody [Yelton et al. (19)] and displayed by 7%-PAGE under reducing and non-reducing conditions a molecular weight of about 52 kD or 58 kD respectively.

Example 3

Characterisation of chimaeric polypeptides

HIV-I (12) infected or uninfected Jurkat cells were cultivated under standard conditions, $^{51}$Cr labelled as known in the art and incubated with a cytotoxic T-cell clone (CD3$^{+}$) (15) in the presence of different concentrations of CD4-FvCD4-Cx. A 5 hour $^{51}$Cr release assay was performed as described by Lanzavecchia and Scheidegger (15) with an E:T ratio of 10. Results of this assay are given in Figure 4.

References

1. Dalgleish, A.G. et al. Nature 321, 763-766(1984).
2. Klatzmann, D. et al. Nature 312, 767-768 (1984).
3. McDougal, J.S. et al. J. Immunol. 135, 3151-3162 (1985).
4. Maddon, P.J. et al. Cell 47, 333-348 (1986).

EP 0 505 908 A1

5. Smith, D.H. et al. Science 238, 1704-1707 (1987).
6. Fisher, R.A. et al. Nature 331, 76-78 (1988).
7. Hussey, R.E. et al. Nature 331, 78-81 (1988).
8. Deen, K.C. et al. Nature 331, 82-84 (1988).
9. Traunecker, A. et al. Nature 331, 84-86 (1988).
10. Chaudhary, V.K. et al. Nature 335, 369-372 (1988).
11. Capon, D.J. et al. Nature 337, 525-531 (1989).
12. Traunecker, A. et al. Nature 339, 68-70 (1989).
13. Maddon et al., Cell 42, 93-104 (1985).
14. "DNA Clonding", Vol. II., ed. by Glover, D.M., IRL Press, Oxford (1985).
15. Lanzavecchia, A. & Scheidegger, D. Eur. J. Immunol 17, 105-111 (1987).
16. Huston, J.S. et al. Proc. Natl. Acad. Sci. USA 85, 5897-5883 (1988).
17. Milstein, C. & Cuello, C.C. Nature 305, 537-539 (1983).
18. Orlandi, R. et al. Proc. Natl. Acad. -Sci. USA 86, 3833-3837 (1989).
19. Yelton, D.E. et al. Hybridoma-1, 5-7 (1981).
20. Villarejo et al. J. Bacteriol. 120, 466-474 (1974).
21. Popovic et al., Science 224, 497-499 (1984).
22. Chomczynski, P. & Sacchi, N. Analytical Biochemistry 162, 156-159 (1987).
23. Köhler, G. et al. Med. Oncol. Tumor Pharmacother 1, 227-231 (1984).
24. Horton, R.M. et al. Gene 77, 61-68 (1989).
25. "Culture of animal cells: A manual of basic technique", Alan R. Liss, Inc., New York (1983).
26. J. Sambrook, E.F., Fritsch and T. Maniatis: Molecular Cloning, A Laboratory Manual, 2nd ed., Cold Spring Harbor Laboratory, Cold Spring Harbor, Cold Spring Harbor Laboratory Press (1989).
27. H. Harlow and D. Lane: "Antibodies, A Laboratory Manual", Cold Spring Harbor Laboratory, Cold Spring Harbor, Cold Spring Harbor Laboratory Press, (1988).
28. Oi, V.T. et al. Proc. Natl. Acad. Sci. USA 80, 825-829 (1983).
29. Ochi, A. et al. Nature 302, 340-342 (1983).
30. Neuberger, M.S. et al. EMBO J. 2, 1373-1378 (1983).
31. Kabat, E.A. et al. "Sequences of Proteins of Immunological Interest" [U.S. Dept. of Health and Human Services, U.S. Government Printing Office, (1987)].
32. Martin, H. et al., Cell 63, 203-211 (1990).
33. Traunecker, A. et al., Eur. J. Immunol. 16, 851-854 (1986).
34. Tal, M.-S. et al., Biochem. 29, 8024-8030 (1990).
35. Namen, A. et al., J. Exp. Med. 167, 988 (1988).

**Claims**

1. A DNA sequence comprising two DNA subsequences, each subsequence-independently from the other one-coding for a ligand binding site of a human receptor, an antigen binding site or a ligand of a human receptor and optionally further comprising a third DNA subsequence coding for the constant domain or parts thereof of an immunglobulin *x* or λ-light chain.

2. A DNA sequence according to claim 1 whereby one of the two DNA subsequences codes for a ligand binding site of a human receptor and the other DNA subsequence codes for an antigen binding site.

3. A DNA sequence according to claim 1 whereby the two DNA subsequences code for antigen binding sites with the same or different antigenic specificity.

4. A DNA sequence according to claim 1 whereby one of the two DNA subsequences codes for a ligand of a human receptor and the other DNA subsequence codes for an antigen binding site.

5. A DNA sequence according to claim 2 whereby the ligand binding site of a receptor is represented by the first two domains of the human CD4 molecule or fragments thereof which still bind the ligand.

6. A DNA sequence according to claim 4 or 5 whereby the antigen binding site recognizes the human CD3 molecule.

7. A DNA sequence according to claim 3 whereby one of the two antigen binding sites recognizes the

11

human CD3 molecule.

**8.** A vector comprising a DNA sequence according to anyone of claims 1-7.

**9.** A vector as claimed in claim 8 which is an expression vector.

**10.** A prokaryotic or eukaryotic host cell transformed with a vector as claimed in claim 8 or 9.

**11.** A polypeptide encoded by a DNA sequence as claimed in anyone of claims 1-7.

**12.** The polypeptide of claim 11 as a therapeutically active agent.

**13.** The polypeptide encoded by a DNA sequence as claimed in claims 5 and 6 as a therapeutically active agent against AIDS.

**14.** A method for the production of a polypeptide of any one of claims 11 to 13 which method comprises culturing in a suitable medium a transformed host cell as claimed in claim 10 and isolating said polypeptide.

**15.** A pharmaceutical composition containing at least one polypeptide as claimed in claim 11 and a therapeutically acceptable carrier material.

**16.** The use of a polypeptide as claimed in anyone of claims 11 to 13 for the preparation of a pharmaceutical composition.

**17.** The use of a polypeptide as claimed in claim 13 for the preparation of a pharmaceutical composition for the treatment of AIDS.

**Claims for the following Contracting States : GR, ES**

**1.** A process for the preparation of a polypeptide encoded by a DNA sequence comprising two DNA subsequences, each subsequence-independently from the other one-coding for a ligand binding site of a human receptor, an antigen binding site or a ligand of a human receptor and optionally further comprising a third DNA subsequence coding for the constant domain or parts thereof of an immunglobulin $x$ or λ-light chain which process comprises culturing in a suitable medium a host cell transformed with a vector capable of expressing said polypeptide and isolating said polypeptide.

**2.** A process according to claim 1 wherein one of the two DNA subsequences codes for a ligand binding site of a human receptor and the other DNA subsequence codes for an antigen binding site.

**3.** A process according to claim 1 wherein the two DNA subsequences code for antigen binding sites with the same or different antigenic specificity.

**4.** A process according to claim 1 wherein one of the two DNA subsequences codes for a ligand of a human receptor and the other DNA subsequence codes for an antigen binding site.

**5.** A process according to claim 2 wherein the ligand binding site of a receptor is represented by the first two domains of the human CD4 molecule or fragments thereof which still bind the ligand.

**6.** A process according to claim 4 or 5 wherein the antigen binding site recognizes the human CD3 molecule.

**7.** A process according to claim 3 wherein one of the two antigen binding sites recognizes the human CD3 molecule.

**8.** A process according to anyone of claims 1-7 wherein the host cell is an eukaryotic cell.

**9.** A process according to claim 8 wherein the host cell is a cell from a myeloma cell line.

10. A process according to claim 9 wherein the myeloma cell line is P3X63Ag8.

11. A process according to anyone of claims 8-10 wherein the vector is a pSV2 derived vector.

12. A process according to claim 11, wherein the pSV2 derived vector is pFvCD3-H$_\gamma$3.

13. A process for the preparation of a pharmaceutical composition which process comprises mixing a polypeptide obtained according to a process as claimed in any one of claims 1-12 and, if desired, one or more other therapeutically active substances with a therapeutically inert carrier material and bringing the mixture into a galenical administration form.

14. A pharmaceutical composition containing a polypeptide obtained according to a process as claimed in any one of claims 1-12 and, if desired, one or more other therapeutically active substances and a therapeutically inert carrier material.

15. The use of a polypeptide obtained according to a process as claimed in any one of claims 1-12 for the manufacture of a pharmaceutical composition.

16. The use of a polypeptide obtained according to a process as claimed in any one of claims 1-12 for the manufacture of a pharmaceutical composition for the treatment of AIDS.

# Figure 1a

# Figure 1b

Fig. 2

## Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CELL<br>vol. 64, no. 5, 8 March 1991, CAMBRIDGE MA, USA<br>pages 1037 - 1046;<br>C. ROMEO ET AL.: 'Cellular immunity to HIV activated by CD4 fused to T cell or Fc receptor polypeptides.'<br>* the whole document *<br>--- | 1-17 | C12N15/62<br>C12N15/12<br>C12N15/13<br>C12N1/21<br>C12N5/10<br>C07K13/00<br>A61K37/02 |
| Y | EP-A-0 308 936 (BRISTOL-MYERS COMPANY)<br>* claims *<br>--- | 1-17 | |
| Y | WO-A-9 100 360 (MEDAREX, INC.)<br>* claims *<br>--- | 1-17 | |
| X,P | EMBO JOURNAL<br>vol. 10, no. 12, December 1991, OXFORD, GB<br>pages 3655 - 3659;<br>A. TRAUNECKER ET AL.: 'Bispecific single chain molecules (Janusins) target cytotoxic lymphocytes on HIV infected cells.'<br>* abstract *<br>--- | 1-17 | **TECHNICAL FIELDS SEARCHED (Int. Cl.5)** |
| X,P | PROCEEDINGS OF THE NATIONAL ACADEMY OF SCIENCES OF USA<br>vol. 88, no. 11, June 1991, WASHINGTON DC, USA<br>pages 4723 - 4727;<br>J. BERG ET AL.: 'Bispecific antibodies that mediate killing of cells infected with human immunodeficiency virus of any strain.'<br>* abstract *<br>----- | 1-17 | C07K<br>C12N |

The present search report has been drawn up for all claims

| Place of search | Date of completion of the search | Examiner |
|---|---|---|
| THE HAGUE | 14 JULY 1992 | NOOIJ F.J.M. |

EPO FORM 1503 03.82 (P0401)